# EUROPÄISCHE PATENTSCHRIFT

(11) **EP 4 335 409 B1**
(45) Veröffentlichungstag und Bekanntmachung des Hinweises auf die Patenterteilung: **21.01.2026**
(21) Anmeldenummer: 22194707.0
(22) Anmeldetag: 08.09.2022
(51) Int. Cl.: A61F 2/00

(54) **VERFAHREN ZUR HERSTELLUNG EINES RÄUMLICHEN CHRURISCHEN NETZES**
METHOD FOR PRODUCING A SPATIAL CURGICAL MESH
PROCÉDÉ DE PRODUCTION D'UN RÉSEAU CHIRURGICAL SPATIALISÉ

(43) Veröffentlichungstag der Anmeldung: 13.03.2024
(73) Patentinhaber: Torunskie Zaklady Materialow Opatrunkowych Spolka Akcyjna, 87-100 Torun (PL)
(72) Erfinder: Sujka, Witold, 95-080 Tuszyn (PL); Karbowski, Krzysztof, 32-083 Szczyglice (PL); Wilgocka, Karolina, 98-240 Szadek (PL); Turlakiewicz, Karolina, 91-157 Lódz (PL); Latanska, Ilona, 94-104 Lódz (PL)
(74) Vertreter: Bischof, Oliver

(56) Entgegenhaltungen:
- EP-B1- 0 746 267
- EP-B1- 1 100 401
- DE-A1- 102013 004 574
- DE-A1- 102013 014 295
- DE-A1- 102014 015 179

## Beschreibung

Die Erfindung betrifft ein Verfahren zur Herstellung eines räumlichen, chirurgischen Netzes zur Versorgung von Leistenbrüchen mit offenen und laparoskopischen Techniken, wobei das Netz aus einem nicht resorbierbaren, flexiblen Material besteht Für die chirurgische Versorgung von Hernien, insbesondere von Bauchbrüchen, sind verschiedene Prothetikprodukte bekannt, deren Hauptaufgabe darin besteht, die infolge von Kollagenstörungen im Organismus geschwächten Muskelstrukturen zu stärken.

Aus dem Patent EP 1 100 401 B1 sind räumliche Netze zur chirurgischen Behandlung von Hernien unterschiedlicher Form bekannt, wobei jede Lösung für die Herstellung eines räumlichen Netzes auf demselben Prinzip basiert: das Netz besteht aus zwei Hauptbestandteilen, nämlich aus einem Flechtenwerk aus einem elastischen, nicht resorbierbaren, synthetischen Material sowie aus einer aus einem resorbierbaren Material gefertigten Ummantelung, welche sich durchlaufend um das Flechtenwerk erstreckt und es in einer festgelegten Lage hält.

Die Krümmung des Netzes kann durch Drähte gewährleistet sein, welche die konvexe Form aufrechterhalten, während in der Ummantelung eine Unterbrechung vorhanden sein kann. Die Leitungen sind senkrecht zueinander in Polrichtungen und um die Ummantelung herum konzentrisch angeordnet.

Aus dem Patent EP 0 746 267 B1 ist ein vorgeformtes chirurgisches Netz zur Reparatur von Muskel- oder Gewebewänden bekannt, das aus einem nicht resorbierbaren, elastischen Material gefertigt ist. Das Netz weist eine erste gekrümmte Oberfläche auf, die wiederum eine kugelförmig geformte Abdeckung und ein konisches Teil aufweist, das sich seitlich vom Umfangsabschnitt der Abdeckung erstreckt und zu einer Spitze hin verjüngt. Die Abdeckung und das konische Teil bilden gemeinsam einen Hohlraum mit einer gekrümmten Innenfläche aus. Eine zweite gekrümmte Oberfläche des Netztes erstreckt sich vom Umfang der ersten gekrümmten Oberfläche und ist durch eine abgerundete Kante mit dieser verbunden. Die Herstellung eines solchen Netzes erfolgt durch Einlegen eines flachen chirurgischen Netzes in eine Einrichtung, die eine gekrümmte Matrize aufweist, welche durch ein erstes, kugelförmiges Teil sowie ein zweites, kegelförmiges, sich zum Ende hin verjüngendes Teil gekennzeichnet ist.

In den oben genannten Patentschriften werden Lösungen beschrieben, die Implantate von gemittelten Ausmaßen darstellen, was lediglich mit einer konventionellen, ungefähren Anpassung des Erzeugnisses an die anatomischen Räume verbunden ist, wodurch der außerhalb des Defekts verbleibende Spielraum möglicherweise nicht ausreichend ist. Dies kann zu einem Wiederauftreten der Hernie oder zur Notwendigkeit einer Fixierung während der Implatation führen.

Weit verbreitete flache Netzerzeugnisse bereiten den Chirurgen aufgrund der Konformationsunterschiede Schwierigkeiten bei ihrer Implantation. Weil das Netz sich faltet und knittert, wird das Halten des Produkts an der richtigen Stelle während der Fixierung schwierig.

Auf dem Markt sind auch chirurgische Implantate erhältlich, die so vorgeformt sind, dass sie sich dem anatomischen Raum der Leistengrube in verschiedenen Formen anpassen.

In ihrem Herstellungsprozess wurden die Ausmasse der Implantate jedoch als Mittelwerte bestimmt, was de facto zu lediglich einer ungefähren Anpassung des Implantats an die anatomische Struktur führt.

Es stellt sich daher die Aufgabe, ein Implantat zu entwickeln, welches die vorgenannten Unzulänglichkeiten deutlich verringern kann. Das Produkt sollte die richtigen mechanischen und strukturellen Parameter sichern, um den mit der Benutzung des Netzes nach seiner Implantation verbundenen Komfort so weit wie möglich zu erhöhen. Das Produkt sollte leicht auflegbar und an die anatomischen Strukturen anpassbar sein.

Die vorliegende Erfindung beschreibt ein Verfahren zur Herstellung eines räumlichen chirurgischen Netzes zur Versorgung von Leistenbrüchen in offenen und laparoskopischen chirurgischen Techniken in einer präperitonealen Position, die in zwei Varianten vorliegt, nämlich einer linken und einer rechten. Eine Form für das räumliche Netz gemäß Erfindung zeichnet sich dadurch aus, dass sie eine toroidale Oberfläche, eine zylindrische Oberfläche mit einer dauerhaften Vertiefung nahe der Formmitte, eine kugelige Oberfläche und eine plane Oberfläche umfasst. Die zylindrische bzw. Walzenoberfläche bildet eine Mulde in der Art einer inneren Nierenkrümmung.

Ein räumliches, erfindungsgemäß hergestelltes Netz besteht aus einem nicht resorbierbaren, elastischen Material, das ein gestrickter oder gewebter Stoff ist. Das räumliche Netz kann aus einem Monofilament- oder Multifilamentgarn hergestellt sein. Das erfindungsgemäß hergestellte Produkt ist in seiner Form und Größe signifikant unterschiedlich, so dass die Bedeckung der beschriebenen Strukturen einen ausreichenden Peripheriebereich außerhalb des Herniendefekts gewährleistet, wodurch das Produkt im Operationsfeld ohne zusätzliche Fixierung gehalten werden kann. Dies gilt für alle Arten von Hernien im Leistenbereich, d. h. für Hernien L1, L2 und L3, F1, F2 und F3, wie auch M1 i M2 in den TAPP- und TEP-Verfahren sowie in dem offenen Stoppa-Verfahren, wie auch für M3-Defekte in den als TAPP+ und TEP+ bezeichneten Verfahren.

Die Autoren der vorliegenden Erfindung stützten sich beim Entwerfen des Implantats und bei der Auswahl einer entgültigen räumlichen Form auf CT-Scans der Leiste bei einer repräsentativen Patientengruppe, wodurch eine präzise Anpassung des Implantats an die anatomischen Strukturen möglich wurde.

Vorzugsweise werden zur Herstellung des Netzes weichmacherfreie Thermoplaste, insbesondere das zur Gruppe der Polyolefine gehörende Polypropylen eingesetzt, welches physiologisch unbedenklich und biologisch inert ist. Normalerweise sind Produkte, wie Formteile aus Polypropylen, transluzent, also milchrig. Nach einer Verstreckung unterhalb der Kristallit-Schmelztemperatur von PP und Erniedrigung des Schmelzpunkts auf ca. 150 °C, geht die Transluzenz in Transparenz bei Erhalt der Steifigkeit über. Nach der Verstreckung steigt die mechanische Festigkeit, das Material dehnt sich weniger. Diese Eigenschaften prädisponieren das Material in Form eines PP-Garns zur Fertigung von chirurgischen Netzen. Darüber hinaus können Polyester und Polyvinylidenfluorid (PVDF) für die Herstellung von chirurgischen Netzen verwendet werden. Auch anderes thermoplastisches Material, nämlich Polyethylen (PE) kann für die Herstellung von chirurgischen Netzen geignet sein, obwohl es in seinen physikalischen und chemischen Eigenschaften nicht an Polypropylen heranreicht.

Ein Netzabschnitt wird auf eine gekrümmte, der menschlichen Leistengegend entsprechend profilierte Musterform gelegt und mittels eines flachen, umlaufenden Andrückelements an die Form gedrückt. An den Formrändern werden Befestigungselemente, wie Metallklammern platziert und das Andrückelement, welches das Netz an die Form drückt, wird festgezogen.

Die Musterform für das räumliche Netz ist aus einem Material hergestellt, welches eine relativ hohe Beständigkeit gegen Verformung, darin thermische, aufweist. Vorzugsweise bestehen diese Elemente aus einer Alu-Legierung, welche für eine Verarbeitung mittels CNC-Verfahrens geeignet ist. Es ist nicht ausgeschlossen, für die Herstellung der Musterform einen kunststoff- oder metallverarbeitenden 3D-Drucker einzusetzen. Als geeigneter, insbesondere fester, steifer und hitzebeständiger Kunststoff (bis 215⁰C) hat sich PES (Polyethersulfon) erwiesen. Überdies können Harze, insbesondere Epoxidharze als Materialien zur Herstellung von Musterform zum Einsatz kommen. Ferner ist es möglich, eine einzige Musterform als Vorlage zur Herstellung mehrerer Formen in Einspritzverfahren zu nutzen.

Die so vorbereitete Musterform mit dem aufgelegten Netz wird für eine Dauer von 6 bis 8 Minuten auf eine Temperatur im Bereich von 140°C - 168°C erhitzt. Danach wird das thermisch stabilisierte Netz aus der Form gelöst. Der nächste Schritt beruht auf Ausschneiden der Ränder des Netzes, welches ein fertiges Produkt darstellt.

Ein erfindungsgemäßes Verfahren zur Herstellung eines räumlichen chirurgischen Netzes sowie ein so hergestelltes Netz gemäß Erfindung werden im Folgenden in Ausführungsbeispielen anhand der Zeichnung näher erläutert.

Die Figuren zeigen:
Fig. 1 - eine Musterform in ihrer linken Version, in einer Draufsicht;
Fig. 2 - eine rechte Version der Musterform gemäß Fig. 1, in einer Draufsicht;
Fig. 3 - die Musterform gemäß Fig. 1, mit aufgelegtem Netz;:
Fig. 4 - die Musterform gemäß Fig. 2, ebenso mit aufgelegtem Netz;
Figuren 5 und 6 - die Musterform mit Netz, in zwei pespektivischen Ansichten;
Fig. 7 - ein räumliches, fertiges Netz nach dem Ausschneiden entlang seines umlaufenden Randes, in einer Draufsicht;
Fig. 8 - das fertige Netz gemäß Fig. 7, in einer perspektivischen Ansicht;
Fig. 9 - eine Draufsicht auf das die Musterform bedeckende Andrückelement;
Fig. 10 - einen schematischen Querschnitt der Musterform und des Andrückelementes.

Gleiche oder ähnliche Elemente können in den nachfolgenden Figuren mit gleichen oder ähnlichen Bezugszeichen versehen sein. Ferner enthalten die Figuren der Zeichnung, der Beschreibung sowie die Ansprüche zahlreiche Merkmale in Kombination. Einem Fachmann ist dabei klar, dass diese Merkmale auch einzeln betrachtet werden oder sie zu weiteren, hier nicht näher beschriebenen Kombinationen, zusammengeführt werden können.

### Beispiel 1.

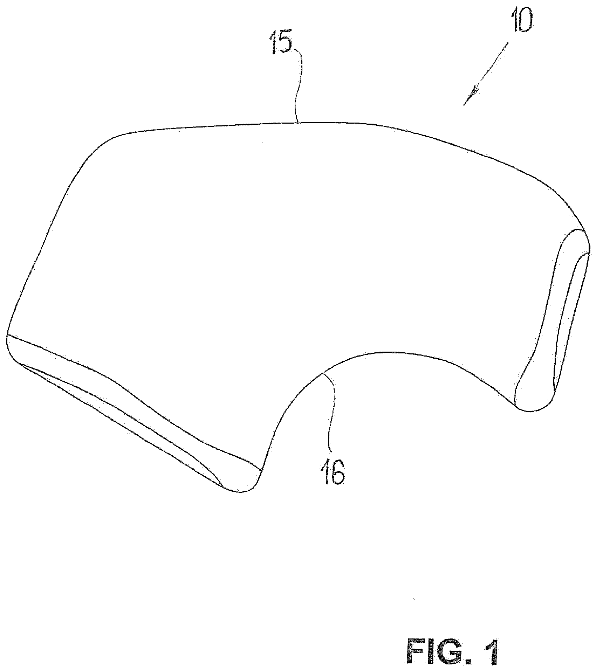

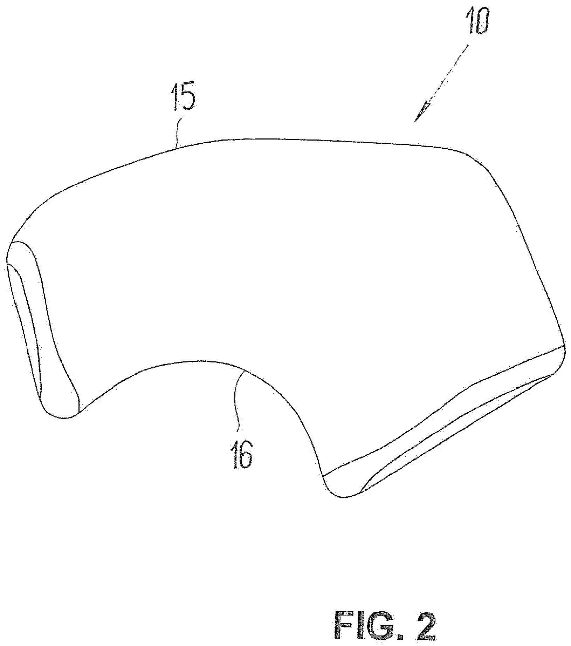

Die Figuren 1 und 2 zeigen eine linke und eine rechte Ausführung einer in CNC-Technik angefertigten Musterform 10, deren Oberfläche entsprechend profiliert ist. Die Musterform 10 stellt ein gekrümmtes Gebilde dar, aufweisend einen konvexen (in Draufsicht) Außenrand 15 und einen dem konvexen abgewandten konkaven Außenrand 15.

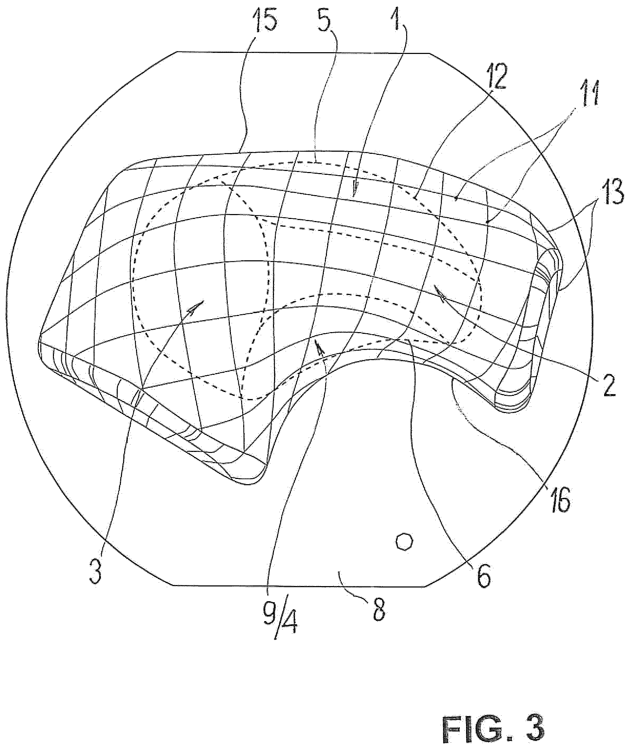

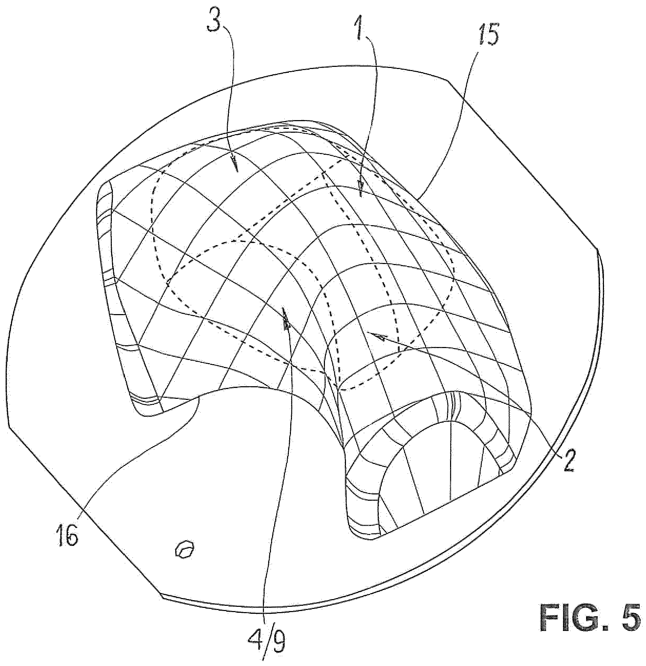

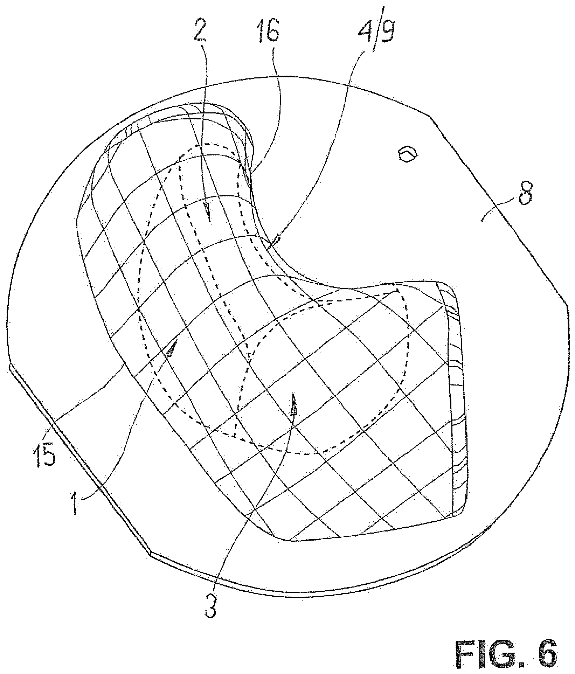

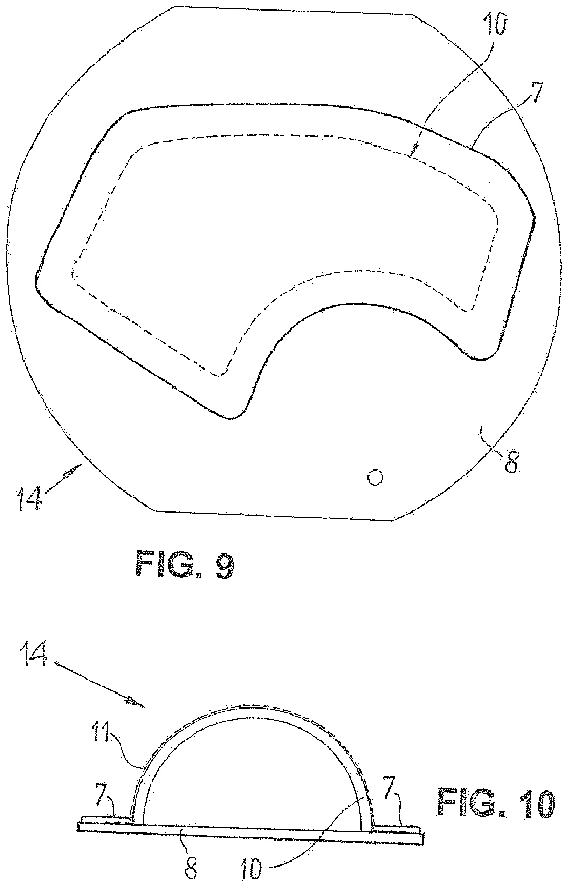

Gemäß Figuren 3 bis 6 ist auf die Oberfläche der Musterform 10 ein Netz 11 dicht aufgelegt. Die dichte Haftung des Netzes 11 wird durch Anpressen einer umlaufenden, flachen Abdeckung 7 (vgl. Fig. 9) mit Hilfe von hier nicht gezeigten Metallklammern erreicht, die auf die Kanten der Musterform 10 aufgesetzt werden. Mit "8" ist eine ebene Arbeitsplatte als Unterlage bezeichnet. Sowohl die Außenfläche der Musterform 10 als auch die Innenfläche des Netzes 11 entsprechen folgenden Teilflächen:
einer toroidalen Oberfläche 2, einer zylindrischen Oberfläche 4, einer kugeligen Oberfläche 3 und einer planen Oberfläche 1. Die zylindrische Oberfläche 4 bildet eine Mulde 9 aus.

Die kugelige Oberfläche 3 des Netzes grenzt an die flache Oberfläche 1, die toroidale Oberfläche 2 und die zylindrische Oberfläche 4 an, wobei die plane Oberfläche 1 an die toroidale Oberfläche 2 angrenzt, welche wiederum an die zylindrische Oberfläche 4 angrenzt.

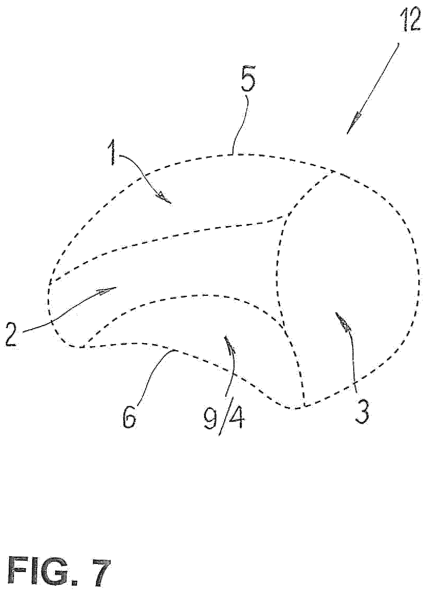

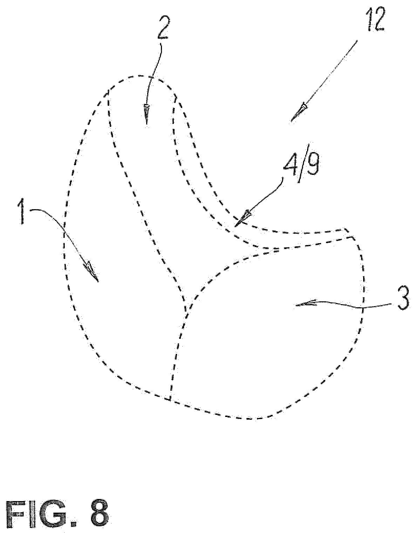

Die gesamte Innenfläche eines einsatzfähigen, fertigen Netzes 12 (alle Krümmungsflächen plus Planfläche, vgl. Figuren 7 und 8) entspricht der gesamten Außenfläche der Musterform 10 im Bereich des Netzrandes 5, 6 und damit der digital ermittelten Form der menschlichen Leistengegend für eine repräsentative Patientengruppe.

Die Ränder des Netzes 5, 6 in Draufsicht auf die profilierte Außenfläche sind etwa U-, nieren- bzw. bumerangförmig.

Die Konturen der Teilflächen 1, 2, 3, 4 wurden der Klarheit der Zeichnung wegen mit Strichlinien gezeigt.

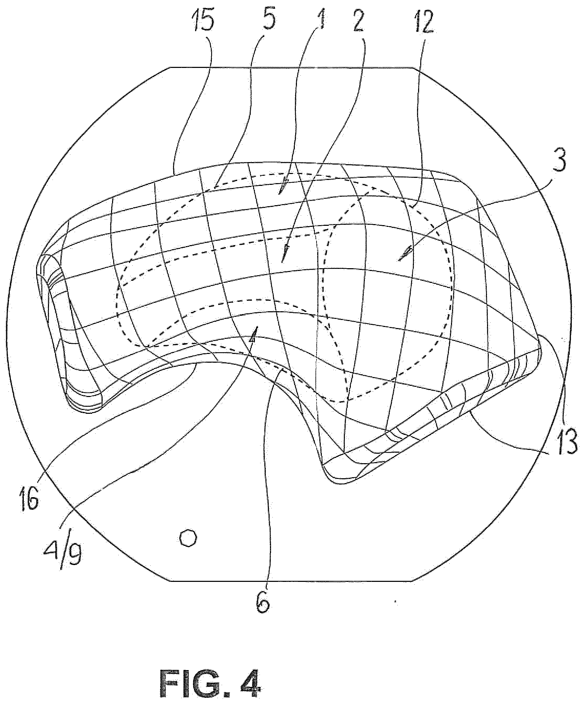

Es wird darauf hingewiesen, dass es sowohl eine linke als auch eine rechte Ausführung (Figuren 3 und 4) des Netzes sowie der Musterform 10 vorgesehen sind.

### Beispiel 2:

Ein räumliches chirurgisches Netz 12 zur Reparatur eines Defekts in der Muskelwand oder im Gewebe eines Patienten wurde auf folgende Weise hergestellt:
Ein flacher Netzabschnitt, hergestellt aus einem glasklaren Polypropylengarn mit einem Durchmesser von 0,16 mm und einem Titer von 185 dtex wurde auf eine gekrümmte, der menschlichen Leistengegend entsprechende Musterform 10 gelegt.

Dann wurden ein die Musterform 10 umgebendes, flaches Andrückelement 7 auf einen Formrand 13 der Musterform 10 so gelegt, dass das Netz 11 mithilfe der Metallklammern fixiert werden kann. Als Formrand 13 soll ein ganzer umlaufende Rand einschließlich Außenrände 15, 16 der Musterform 10 verstanden werden. Das umlaufende Andrückelement 7 wurde also über das Netz 11 an der Musterform 10 festgezogen. Die Musterform 10 ruht mit ihrem unteren umlaufenden Formrand 13 auf der planen Unterlage (Arbeitsplatte 8). Auf dieser Weise wird ein Satz 14 (vgl. Fig. 9) zur Bildung von 3D-Netzen gefertigt.

Als Material zur Anfertigung der Musterform 10 wird eine Aluminiumlegierung gewählt, die sich relativ gut in einem maschinellen CNC-Verfahren verarbeiten lässt.

Die so vorbereitete Musterform 10 mit dem aufgelegten Netz 11 und dem umlaufenden Andrückelement 7 wurde in einem bis auf 155°C erhitzten Trocknungsstabilisator platziert.

Ab dem Moment der Wiedererreichung von 155°C im Inneren des Trocknungsstabilisators wurden 8 Minuten abgezählt.

Das vorher flache Netz nimmt infolge der Krafteinwirkung und Verstreckung eine in Figuren 3 und 4 gezeigte Form an, d. h. es reicht bis zum unteren Formrand 13 hinein.

Danach wurde das thermisch stabilisierte Netz nach einer Selbstkühlung bis zur Umgebungstemperatur (Zimmertemperatur) aus der Musterform 10 gelöst, worauf das Netz einem Konfektionierungsprozess unterzogen wurde, um ihm seine endgültige Form (fertiges Netz 12) zu verleihen.

### Beispiel 3:

Ein räumliches chirurgisches Netz zur Reparatur eines Defekts in der Muskelwand oder im Gewebe eines Patienten wurde auf folgende Weise hergestellt:
Ein flaches, aus einem Polypropylengarn hergestelltes Netz mit einem Durchmesser von 0,10 mm und einem Titer von 72 dtex in einer transparenten Farbe auf eine gekrümmte, der menschlichen Leistengegend entsprechende Musterform 10 gelegt wurde, welche die Form des fertigen Netzes nachbildet.

Anschließend wurde ein umlaufendes, flaches Andrückelement 7 auf dem Formrand 13 der Musterform 10 platziert und an dem Formrand 13 wurden Metallklammern eingebracht. Das Andrückelement 7 wurde an der Musterform 10 festgezogen. Die so vorbereitete Musterform 10 mit dem aufgelegten Netz 11 wurde in einem bis auf 140°C erhitzten Trocknungsstabilisator platziert. Ab dem Moment der Wiedererreichung von 140°C in Inneren des Trocknungsstabilisators wurden 8 Minuten abgezählt. Danach wurde das thermisch stabilisierte Netz aus der Musterform 10 gelöst, worauf das Netz 11 einem Konfektionierungsprozess unterzogen wurde, um ihm seine endgültige Form (fertiges Netz 12 durch Schneiden entlang des Netztrandes 5, 6) zu verleihen. Die Ränder 5, 6 des Netzes 12 sowie der einzelnen profilierten Teilflächen 1, 2, 3, 4 sind mit Strichlinien (Figuren 3 bis 8) dargestellt.

### Beispiel 4:

Ein räumliches chirurgisches Netz zur Reparatur eines Defekts in der Muskelwand oder im Gewebe eines Patienten wurde auf folgende Weise hergestellt:
Ein flaches, aus einem Polypropylengarn hergestelltes Netz mit einem Durchmesser von 0,16 mm und einem Titer von 185 dtex in einer transparenten Farbe wurde auf eine gekrümmte, der menschlichen Leistengegend entsprechende Musterform 10 gelegt, welche die Form des späteren, fertigen Netzes 12 nachbildet.

Dann wurde das umlaufende, flache Andrückelement 7 auf die Musterform 10 gelegt und an den Rändern der Musterform 10 wurden Metallklammern angebracht. Das Netz 11 wurde durch das Andrückelement 7 an der Musterform 10 festgezogen.

Die Musterform 10, das durch das Andrückelement 7 festgezogenes Netz 11 bilden zusammen mit der Arbeitsplatte 8 und den nicht gezeigten Metallklammern einen kompletten Satz 14, welcher sehr schematisch, ohne die profilierten Teilflächen zu zeigen, in Fig. 10 dargestellt ist.

Der so vorbereitete Satz 14 mit der Musterform 10 und dem aufgelegten Netz 11 wurde in einem bis auf 168°C erhitzten Trocknungsstabilisator platziert. Ab dem Moment der Wiedererreichung von 168°C im Inneren des Trocknungsstabilisators wurden 6 Minuten abgezählt. Danach wurde das thermisch stabilisierte Netz aus der Musterform 10 gelöst, worauf das Netz einem Konfektionierungsprozess unterzogen wurde, um ihm seine endgültige Form (fertiges räumliches Netz 12) zu verleihen.

### Bezugszeichenliste:

- 1: plane Oberfläche
- 2: toroidale Oberfläche
- 3: kugelige Oberfläche
- 4: zylindrische Oberfläche
- 5: Netzrand
- 6: Netzrand
- 7: Andrückelement
- 8: Arbeitsplatte
- 9: Mulde
- 10: Musterform
- 11: ganzes Netz
- 12: fertiges Netz (ausgeschnitten)
- 13: Formrand
- 14: Satz
- 15: Außenrand
- 1 6: A u ß e n r a n d (k ü r z e

## Patentansprüche

1. Verfahren zur Herstellung eines räumlichen, chirurgischen Netzes zur Versorgung von Leistenbrüchen, welches aus einem nicht resorbierbaren, flexiblen Material besteht, **dadurch gekennzeichnet, dass** ein flacher Abschnitt eines chirurgischen Netzes auf eine gekrümmte, der menschlichen Leistengegend entsprechende Musterform gelegt wird, welche folgende Teilflächen umfasst:
- eine toroidale Oberfläche (2),
- eine zylindrische Oberfläche (4),
- eine kugelige Oberfläche (3) und
- eine ebene Oberfläche (1),
- wobei die zylindrische Oberfläche (4) eine Mulde (9) bildet,
- wobei die kugelige Oberfläche (3) an die flache Oberfläche (1), die toroidale Oberfläche (2) und die zylindrische Oberfläche (4) angrenzt,
- wobei die flache Oberfläche (1) auf der toroidalen Oberfläche (2) aufliegt, welche wiederum auf der zylindrischen Oberfläche (4) aufliegt,
- danach ein Andrückelement (7) auf der Musterform mit dem Netz sowie Metallklammern an den Rändern der Form platziert werden, und das Andrückelement, welches das Netz an die Musterform drückt, festgezogen wird,
wonach das so platzierte Netz für eine Dauer von 6 bis 8 Minuten auf eine Temperatur im Bereich von 140°C - 168°C erhitzt wird,
und danach das thermisch stabilisierte Netz aus der Musterform gelöst wird und die Ränder des Netzes (5, 6) zur Erzielung eines fertigen räumlichen Netzes (12) geschnitten werden.

2. Verfahren nach Anspruch 1, **dadurch gekennzeichnet, dass** das Netz aus einem nicht resorbierbaren, elastischen Material hergestellt ist, das ein gestrickter oder gewebter Stoff ist.

3. Verfahren nach Anspruch 1 oder 2, **dadurch gekennzeichnet, dass** das nicht resorbierbare Material biologisch inert ist.

4. Verfahren nach Anspruch 3, **dadurch gekennzeichnet, dass** der gestrickte oder gewebte Stoff aus einem Monofilament- oder Multifilamentgarn, vorzugsweise Polypropylengarn hergestellt ist.

5. Verfahren nach einem der Ansprüche 1 bis 4, **dadurch gekennzeichnet, dass** die der menschlichen Leistengegend entsprechende, räumliche Musterform (10) der Leiste mit Hilfe der Computertomographie bei einer repräsentativen Patientengruppe digital berechnet wird.

6. Verfahren nach Anspruch 5, **dadurch gekennzeichnet, dass** die ermittelte Musterform (10) der Leiste als Datensatz auf eine numerische CNC-Werkzeugmaschine oder einen 3D-Drucker übertragen wird.

## Claims

1. A method for producing a spatial surgical mesh for treating hernias, the mesh consisting of a non-resorbable, flexible material, **characterized in that** a flat section of a surgical mesh is laid onto a curved pattern form corresponding to the human groin area, the pattern form comprising the following partial surfaces:
- a toroidal surface (2),
- a cylindrical surface (4),
- a spherical surface (3) and
- a flat surface (1),
- wherein the cylindrical surface (4) forms a trough (9),
- wherein the spherical surface (3) borders the flat surface (1), the toroidal surface (2), and the cylindrical surface (4),
- wherein the flat surface (1) lies on the toroidal surface (2) which, in turn, lies on the cylindrical surface (4),
- a pressure element (7) is then placed on the pattern form with the mesh, and metal clips are placed on the edges of the form, and the pressure element, which presses the mesh against the pattern form, is tightened,
whereupon the mesh so placed is heated to a temperature in the range from 140°C - 168°C for a duration of 6 to 8 minutes,
and then the thermally stabilized mesh is detached from the pattern form, and the edges of the mesh (5, 6) are cut to achieve a finished spatial mesh (12).

2. The method according to claim 1, **characterized in that** the mesh is produced from a non-resorbable, elastic material that is a knitted or woven substance.

3. The method according to claim 1, or 2, **characterized in that** the non-resorbable material is biologically inert.

4. The method according to claim 3, **characterized in that** the knitted or woven substance is produced from a monofilament or multi-filament yarn, preferably a polypropylene yarn.

5. The method according to one of claims 1 to 4, **characterized in that** the spatial pattern form (10) of the groin corresponding to the human groin area is digitally calculated for a representative patient group with the aid of computer tomography.

6. The method according to claim 5, **characterized in that** the determined pattern form (10) of the groin is transferred as a dataset to a numerical CNC machine tool or to a 3D printer.

## Revendications

1. Procédé de fabrication d'un filet chirurgical spatial pour le traitement des hernies inguinales, qui est constitué d'un matériau flexible non résorbable, **caractérisé en ce qu'**une section plate d'un filet chirurgical est placée sur une moule incurvée, correspondant à la région inguinale humaine, qui comprend les surfaces partielles suivantes :
- une surface toroïdale (2),
- une surface cylindrique (4),
- une surface sphérique (3) et
- une surface plane (1),
- dans lesquelles la surface cylindrique (4) forme une gouttière (9),
- dans lesquelles la surface sphérique (3) borde la surface plane (1), la surface toroïdale (2) et la surface cylindrique (4),
- dans lesquelles la surface plane (1) repose sur la surface toroïdale (2), qui repose elle-même sur la surface cylindrique (4),
- après un élément de pression (7) ainsi que des clips métalliques sont placés sur les bords de la moule avec le filet et l'élément de pression, qui plaque le filet contre la moule, est serré à bloc,
après le filet ainsi placé est ensuite chauffé à une température comprise dans la plage de 140°C jusqu'à 168°C pendant une période de 6 à 8 minutes,
et puis le filet thermiquement stabilisé est retiré de la moule et les bords du filet (5, 6) sont coupés pour obtenir un filet spatial fini (12).

2. Procédé selon la revendication 1, **caractérisé en ce que** le filet est fabriqué d'un matériau élastique non absorbant, qui est un tissu tricoté ou tissé.

3. Procédé selon la revendication 1 ou 2, **caractérisé en ce que** le matériau non résorbable est biologiquement inerte.

4. Procédé selon la revendication 3, **caractérisé en ce que** le tissu tricoté ou tissé est fabriqué à partir d'un fil monofilament ou multifilament, de préférence un fil de polypropylène.

5. Procédé selon une des revendications 1 à 4, **caractérisé en ce que** la moule spatiale (10) de l'aine correspondant à la région inguinale humaine est calculée numériquement à l'aide d'une tomographie informatisée dans un groupe représentatif de patients.

6. Procédé selon la revendication 5, **caractérisé en ce que** la moule déterminée (10) de l'aine est transféré sous forme d'un ensemble de données à une machine-outil à commande numérique CNC ou à une imprimante 3D.
